# EUROPEAN PATENT SPECIFICATION

(11) **EP 3 367 981 B1**
(45) Date of publication and mention of the grant of the patent: **04.01.2023**
(21) Application number: 16794147.5
(22) Date of filing: 27.10.2016
(51) Int. Cl.: A61F 5/01

(54) **HINGE ASSEMBLY FOR AN ORTHOPEDIC DEVICE**
SCHARNIERANORDNUNG FÜR EINE ORTHOPÄDISCHE VORRICHTUNG
ENSEMBLE DE CHARNIÈRE POUR DISPOSITIF ORTHOPÉDIQUE

(30) Priority: 30.10.2015 US 201562248541 P
(43) Date of publication of application: 05.09.2018
(73) Proprietor: Ossur Iceland EHF, 110 Reykjavik (IS)
(72) Inventor: HSU, Henry, Foothill Ranch, California 92610 (US); DUNN, Adam, Foothill Ranch, California 92610 (US)
(74) Representative: DenK iP bv
(86) International application number: PCT/US2016/059005
(87) International publication number: WO 2017/075143

(56) References cited:
- US-A- 4 732 143
- US-A- 4 791 916
- US-A- 6 001 075
- US-A1- 2010 049 108

## Description

### TECHNICAL FIELD

The disclosure relates to a hinge assembly for use with an orthopedic or prosthetic device.

### BACKGROUND

Many orthopedic devices include hinges that support joints, and control and limit joint movements. Such joints can include the knee, elbow, shoulder, hip, ankle and wrist joints.

The knee joint comprises two joints, lateral and medial, between the femur and tibia, and one arthrodial joint between the patella and femur. The primary movements of the knee comprise flexion (i.e., rearward rotational movement of the tibia relative the femur), and extension (i.e., forward rotational movement of the tibia relative the femur).

The flexion and extension movements of the knee joint are not pivotal movements about a fixed axis. During flexion, the axis around which movement takes place shifts backward, and during extension it shifts forward. This differs from a more typical hinge joint, such as an elbow, where the axis of rotation does not shift. As full extension is reached, the tibia is rotated inward or rearward, and the joint is disposed in a "locked" position with the ligaments taut. This gives the joint greater stability in the extended position. As flexion is initiated, the tibia initially lowers or moves downwardly with small external rotation of the tibia unlocking the joint and subsequently the tibia rotates or rolls about the joint to full flexion. The initial unlocking of the knee joint during flexion precedes actual full rotation of the knee.

Because of the complexity associated with knee movement, a knee brace hinge must be able to simulate the anatomical movements of the knee. Incorporating such movement into the hinge is crucial, as the knee brace must optimally support the knee joint of its user. The knee brace hinge can also be designed to assist in applying loads to the knee that will improve in healing of injuries.

Many attempts have been made to make a knee brace hinge that is both anatomical and robust, yet such attempts have resulted in hinges that are relatively complex, expensive, and limited in control and movement. For instance, known geared hinges are robust and easily manufactured but produce only one motion relationship that is neither anatomical nor provides loads to heal a knee injury. Other known hinges such as traditional 4-bar hinges may produce a more natural knee movement but are very complicated in design and provide limited control over the motion of the knee brace.

US 4791916 discloses a brace for a knee, utilizing a flexible cage tightly wrapped about the knee, from which a brace structure is suspended and utilizing polycentric hinges at cage lateral regions including arms having controlled ranges of pivotal movement. Upper hinge arms are attached to a cup embracing the thigh and lower hinge arms attach to a cuff affixed to the lower leg. Straps drawing the cage tightly to the knee affixed to the cuffs suspend the brace from the cage, and the polycentric hinges accurately control the extent of leg flexion and extension. The hinge arms are interconnected through a gear and rack relationship, and stops associated with the rack limit arm movement.

US 4732143 describes a hinge for a knee brace or other hinged device, having a selectable extension stop for limiting the pivotal movement allowed by the hinge.

There is a need for a hinge assembly that is both easily manufactured and robust, and capable of producing different motion relationships and loading profiles on a knee brace.

US 6 001 075 is seen as the closest prior art document and discloses a dynamic splint for a joint of a limb undergoing rehabilitation. The splint having a pair of elements each comprising a first tubular strut, a second lever-like strut and an intermediate gear housing mounted on one end of the first strut.

### SUMMARY

Hinge assembly embodiments described herein are adapted to imitate anatomical knee motion and/or create desired loads on the knee in a simpler and more effective way than in the prior art. It will be appreciated that the hinge assembly can be adapted for use with orthopedic devices and/or prosthetic devices.

The present invention relates to a method as set out in the claims.

The first and second profiles can be selected or varied in any way needed to allow the second hinge arm to travel along the defined motion path. The defined motion path can include a complex hinge motion. A complex hinge motion is when at least one of the hinge arms translate relative to the other in a non-linear or variable manner. For instance, a complex hinge motion can include, for example, translation of the second hinge arm relative to the first hinge arm to generally match anatomical knee motion and/or to apply loads to the knee. Moreover, the interaction between the teeth of the first and second gears provides robust control over the relative motion between the hinge arms, allowing for many functional designs. In addition, hinge assembly embodiments according to the present disclosure allow for a simpler construction than the 4-bar linkage hinges of the prior art, making them easier and more intuitive to design.

### BRIEF DESCRIPTION OF THE DRAWINGS

These and other features, aspects, and advantages of the present disclosure will become better understood regarding the following description, appended claims, and accompanying drawings.
Fig. 1 is a schematic view of a hinge assembly according to an embodiment in a first position.
Fig. 2 is a schematic view of the hinge assembly in Fig. 1 showing its instantaneous center of rotation curve and with the linkage and rotation stop removed for ease of reference.
Fig. 3 is a schematic view of the hinge assembly in Fig. 2 in a second position.
Fig. 4 is a schematic view of a prior art hinge assembly.
Fig. 5 is a schematic view of a hinge assembly according to an embodiment in reference to a prior art hinge assembly.
Fig. 6 is an overview of the steps in an embodiment of making a hinge assembly.
Fig. 7 is a schematic view of a hinge assembly according to another embodiment in a first position.
Fig. 8 is a schematic view of the hinge assembly in Fig. 7 in a second position.
Fig. 9 is a schematic view of a hinge assembly according to another embodiment in a first position.
Fig. 10 is a schematic view of the hinge assembly in Fig. 9 in a second position.
Fig. 11 is a schematic view of a hinge assembly according to another embodiment in a first position.
Fig. 12 is a schematic view of the hinge assembly in Fig. 11 in a second position.
Fig. 13 is a graph depicting the horizontal displacement of some of the hinge assembly embodiments relative to a traditional polycentric hinge and a traditional 4-bar hinge.
Fig. 14 is a graph depicting the vertical displacement of some of the hinge assembly embodiments relative to a traditional polycentric hinge and a traditional 4-bar hinge.

### DETAILED DESCRIPTION OF VARIOUS EMBODIMENTS

A better understanding of different embodiments of the disclosure may be had from the following description read with the accompanying drawings in which like reference characters refer to like elements.

Numerous hinge assembly embodiments are described herein, with particular focus given to the knee joint and surrounding areas. The hinge assembly embodiments may serve in protective, preventative or remedial capacities. While the hinge assembly is described within the context of a preferred embodiment that is directed to treating the knee, many of the features described herein may be extended to orthopedic devices and components that secure other joints and body parts. Thus it should be appreciated that the hinge assembly embodiments may be dimensioned to accommodate different types, shapes and sizes of human joints and appendages. It should also be appreciated that the hinge assembly can be adapted for use in prosthetic devices.

The terms "rigid" and "flexible" may be used herein to distinguish characteristics of portions of the hinge assembly. The term "rigid" is intended to denote that the frame is generally devoid of flexibility. Within the context of frame members that are "rigid," it is intended to indicate that they may break if bent with sufficient force. On the other hand, the term "flexible" is intended to denote that features are capable of repeated bending. The term "resilient" may be used to qualify such flexible features as generally returning to the initially molded shape without permanent deformation.

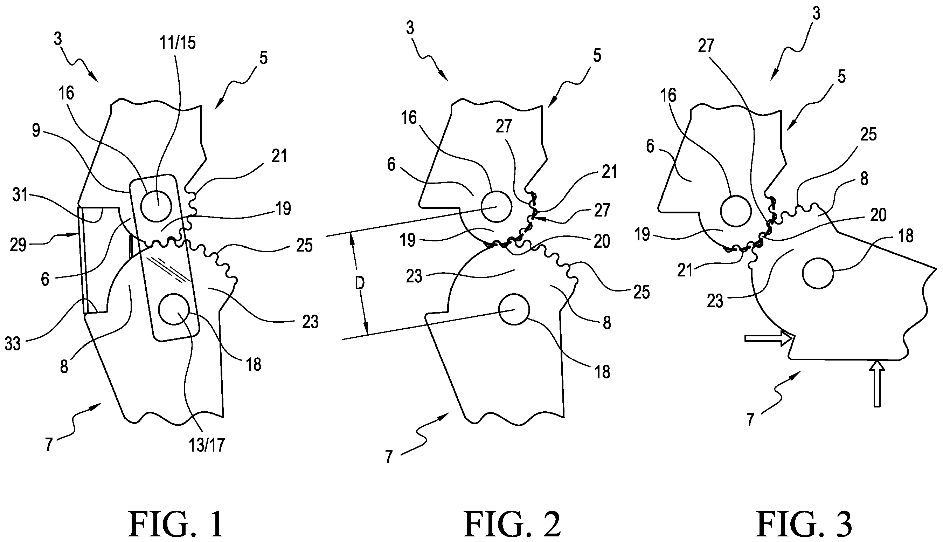

Figs. 1-3 show various views of a hinge assembly 3 for use in an orthopedic device such as a hinge of a knee brace according to an embodiment. The hinge assembly 3 includes a first or upper hinge arm 5 and a second or lower hinge arm 7. A linkage 9 is pivotally attached at a first location point 11 to the upper hinge arm 5 and at a second location point 13 to the lower hinge arm 7. The linkage 9 can comprise a center linkage.

The connection of the linkage 9 at the second location point 13 may rotate around the first location point 11 so that when the hinge assembly 3 moves between flexion and extension, the lower hinge arm 7 and the linkage 9 move around the first location point 11 and peripheral edges of the upper hinge arm 5. Each of the hinge arms 5, 7 and linkage 9 may be formed of metal, carbon fiber, plastic, or any other material which would provide sufficient strength to resist undesirable deformation during use.

According to a variation, a distal portion 6 of the upper hinge arm 5 defines at least one first through-hole 15 generally corresponding to the first location point 11, and arranged to receive at least one first fastener 16 (e.g., a rivet) for connecting the linkage 9 to the upper hinge arm 5. At least one second through-hole 17 can be defined in a proximal portion 8 of the lower hinge arm 7 that generally corresponds to the second location point 13, and is arranged to receive at least one second fastener 18 for connecting the linkage 9 to the lower hinge arm 7. The first and second through-holes 15, 17 can be spaced from one another a distance D. The distance D can be selected so that teeth of the upper and lower hinge arms described below mesh with one another during use.

The distal portion 6 of the upper hinge arm 5 defines a first gear 19 having a first plurality of teeth 21 along at least a portion of its periphery. The proximal portion 8 of the lower hinge arm 7 defines a second gear 23 having a second plurality of teeth 25 along at least a portion of its periphery. The second teeth 25 of the second gear 23 are arranged to interact or mesh with the first teeth 21 of the first gear 19. This beneficially helps control or limit flexion and/or extension of the hinge assembly 3 in a relatively simple manner. For instance, the limits of the periphery of the first gear 19 defining the first teeth 21 can provide an extension or flexion stop to relative movement between the upper hinge arm 5 and the lower hinge arm 7. The first and second teeth 21, 25 can have any suitable shape but are shown including generally rounded sides.

In use, the upper hinge arm 5 is generally fixed relative to a user's femur, and the lower hinge arm 7 rotates and translates relative to the upper hinge arm 5 during gait. The movement of the lower hinge arm 7 about the upper hinge arm 5 is based at least in part on the force applied to a distal portion of the lower hinge arm 7 during gait relative to an instantaneous center of rotation ("ICoR") 20 for the lower hinge arm 7. Generally, the ICoR 20 of the lower hinge arm 7 is a point about which the lower hinge arm 7 rotates as the hinge assembly 3 moves between extension and knee flexion.

The location of the ICoR 20 of the lower hinge arm 7 can depend on the relative sizes, profiles, and/or arrangement between the first and second gears 19, 23 and the position of the first and second teeth 21, 25. In use, the ICoR 20 of the lower hinge arm 7 shifts as the user's knee moves through extension and flexion because the point of contact between the first teeth 21 and second teeth 25 moves as the lower hinge arm 7 rotates relative to the upper hinge arm 5. The ICoR 20 moves along an ICoR path 27. An ICoR path is generally a path along which an ICoR of an object moves while the object is rotating. As the ICoR 20 of the lower hinge arm 7 shifts, the position of the lower hinge arm 7 relative to the upper hinge arm 5 shifts, resulting in translational movement of the lower hinge arm 7 as it rotates.

The profiles of the first and second gears 19, 23 along the first and second teeth 21, 25 at least in part defines the movement of the ICoR 20 of the lower hinge arm 7 relative to the upper hinge arm 5. As such, the profiles of the first and second gears 19, 23 along the first and second teeth 21, 25 can be selected or varied as needed to match a defined motion path including a complex hinge motion (e.g., when at least one of the hinge arms translate relative to the other in a non-linear or variable manner). This beneficially allows the hinge assembly 3 to be adapted to imitate anatomical knee motion and/or apply loads on the knee that may improve in healing of injuries or correct deformities. This is advantageous over traditional geared hinges, which produce only one simple motion relationship that is neither anatomical nor provides loads to heal a knee injury.

In the illustrated embodiment, the first profile of the first gear 19 generally follows a segment of a circle and is larger than the second profile of the second gear 23, which also generally follows a segment of a circle. As the lower hinge arm 7 rotates about the upper hinge arm 5, the ICoR 20 of the lower hinge arm 7 moves along the ICoR path 27 as seen in Figs. 2 and 3. The ICoR path 27 is shown extending along an arcuate line through the first teeth 21 of the first gear 19, generally corresponding to the first profile. It will be appreciated that the first and second profiles of the gears in the hinge assembly embodiments can vary together such that at any specific or particular rotational position of the lower hinge arm relative to the upper hinge arm, the radius of the first gear plus the radius of the second gear is generally constant.

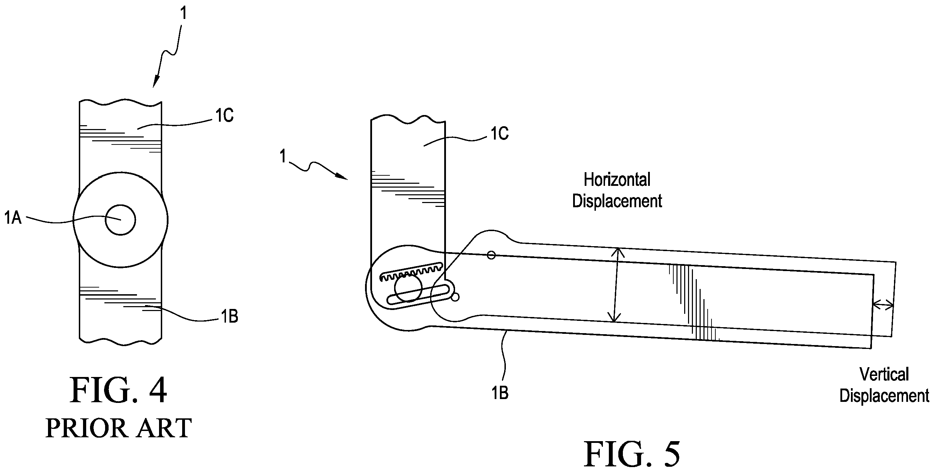

In this discussion, the term "vertical" is defined as a direction along the tibial axis, while the term "horizontal" is defined as a direction perpendicular to the tibial axis in the sagittal plane. It should be appreciated that the terms vertical and horizontal can be relative to any corresponding limb and are not necessarily limited to the leg or in a fixed Cartesian space. Referring briefly to Figs. 4 and 5 for context, measurements provided herein are generally in reference to a traditional single point hinge 1, where the ICoR 1A of its lower hinge arm 1B is fixed. There is no ICoR path because the ICoR 1A does not move. There is no vertical or horizontal translation when the lower hinge arm 1B rotates relative to an upper hinge arm 1C. As shown in Fig. 5, vertical shifting away from the knee center is positive. Horizontal shifting towards the femoral frame is positive.

Referring again to Figs. 2 and 3, when the hinge assembly 3 moves from extension toward flexion (full flexion being at least 90 degrees), the interaction between the teeth 21, 25 along the first and second profiles of the first and second gears 19, 23 shifts the lower hinge arm 7 in both a vertical direction and a horizontal direction relative to the upper hinge arm 5, producing a translational movement of the lower hinge arm 5 in the hinge assembly 3. The lower hinge arm 7 is shown shifting relative to the upper hinge arm 5 in a positive vertical direction and a negative horizontal direction at about 90 degrees flexion.

As the user's knee rotates back toward extension or past a set flexion angle, the contact or interaction between the teeth 21, 25 along the profiles of the first and second gears 19, 23 can move the lower hinge arm 7 horizontally and/or vertically back toward its original position.

The movement of the lower hinge arm 7 relative to the upper hinge arm 5 is defined or limited by the interaction between the first and second teeth 21, 25. As such, the hinge assembly 3 is more robust and provides for greater control over relative motion of the hinge arms 5, 7 than prior art 4-bar linkage hinges, allowing for more versatile functional designs.

As discussed above, the ICoR 20 of the lower hinge arm 7 is generally located at the point of contact between the first and second gears 19, 23 along the first teeth 21 on the upper hinge arm 5. Because the ICoR is at or near the periphery of the lower hinge arm 7, the translational movement of the lower hinge arm 7 can be limited for each degree of rotation. For instance, there may be a horizontal shifting of the lower hinge arm 7 of about 1 millimeter (mm) during a specified range of flexion or extension. The desired shifting may vary from patient to patient, in that one patient may require 3 mm of shifting whereas another patient having different anatomical proportions may require 5 mm of shifting. The orientation and profiles of the first and second gears 19, 23 can be arranged accordingly.

The profiles of the first and second gears 19, 23 along the first and second teeth 21, 25 may have a variety of arrangements, and may be configured to adjust the ICoR path 27 of the lower hinge arm 7. The adjustment of the ICoR path 27 may result in different motions or movements of the lower hinge arm 7 relative to the upper hinge arm 5. Thus, by way of example, by selectively varying the profiles of the gears 19, 23 along the teeth 21, 25 relative to one another, the hinge assembly 3 can be adjusted or adapted to imitate the ICoR behavior of the biological knee, allowing the hinge assembly 3 to comfortably and naturally support the knee in a simpler and more robust manner than in more complicated hinges of the prior art.

By way of another example, the hinge assembly 3 can be specifically adapted to deviate the motion of the lower hinge arm 7 from anatomical knee motion. For instance, the profiles of the gears 19, 23 along the teeth 21, 25 can be varied relative to one another to create a misalignment between the motion of the hinge assembly 3 and the biological knee during flexion and/or extension, which, in turn, can be used to generate forces to supplement and/or support an injured ligament during use of the hinge assembly 3 with a knee brace.

The hinge assembly 3 can thus be arranged and/or adapted to generate specific or complex movements in a simple and robust design.

According to a variation, the distal portion 6 of the upper hinge arm 5 can have an irregular geometric shape along its anterior side, and an increased width relative to other portions of the upper hinge arm 5. This has the effect of stiffening the distal portion 6 of the upper hinge arm 5, which helps the hinge assembly 3 resist twisting during use. The proximal portion 8 of the lower hinge arm 7 can also have an irregular shape and/or increased width to help stiffen the proximal portion 8 of the lower hinge arm 7. In addition, the proximal portion 8 of the lower hinge arm 7 can be wider than the distal portion 6 of the upper hinge arm 5 to provide a larger rotational radius.

Optionally, the hinge assembly 3 can include one or more rotation stops arranged to limit rotation of the hinge arms 5, 7 at a predetermined angle. For instance, a rotation stop 29 can be disposed on the anterior side of the hinge assembly 3 between the upper and lower hinge arms 5, 7. The anterior side of the distal portion 6 of the upper hinge arm 5 can define a stop surface 31 that generally complements or corresponds to an upper surface of the rotation stop 29 and the anterior side of the proximal portion 8 of the lower hinge arm 7 can define a stop surface 33 that generally complements or corresponds to a lower surface of the rotation stop 29. By way of example, the limiting of rotation can be achieved when the hinge arms 5, 7 abut the rotation stop 29 in knee extension. It will be appreciated that the rotation stop 29 can have any suitable shape and construction.

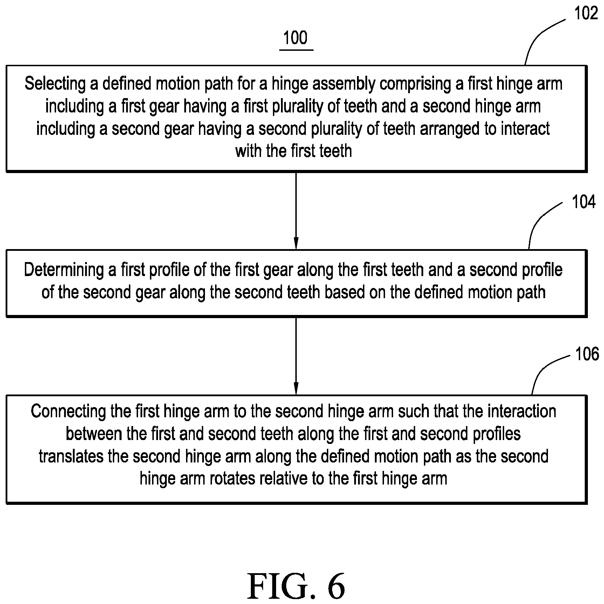

One will appreciate that embodiments of the present disclosure can also be described in terms of flowcharts including one or more steps for accomplishing a particular result. For example, the steps in Fig. 6 and the corresponding text describes steps in a method for making a hinge assembly according to an embodiment.

For instance, Fig. 6 illustrates a method 100 in accordance with the present disclosure for making a hinge assembly which includes step 102 of selecting a defined motion path of a hinge assembly. The hinge assembly can comprise a first hinge arm including a first gear having a first plurality of teeth, and a second hinge arm including a second gear having a second plurality of teeth arranged to interact with the first teeth. The defined motion path can include a complex hinge motion where at least one of the hinge arms translates relative to the other in a non-linear or variable manner. Step 102 can include selecting a defined motion path to generally match anatomical knee motion. Step 102 can include selecting a defined motion path as part of a treatment protocol. Step 102 can include selecting a defined motion path to apply loads on a user's knee that may improve the healing of injuries. Step 102 can include selecting a defined path to apply loads on a user's knee that can help correct deformities. Step 102 can include selecting a defined path to improve user comfort.

Fig. 6 also shows that the method can include the step 104 of determining a first profile of the first gear along the first teeth and a second profile of the second gear along the second teeth based on the defined motion path.

Step 104 can include selecting hinge arms from an inventory of hinge arms having different profiles along their teeth. Inventories, as used herein, may include collections or kits of immediately available components, or the inventory may have on-demand nature, such that when a desired hinge arm is not immediately available, it can nevertheless be ordered as needed. Hinge arms in the inventory can vary in material, gear profiles, size, and/or shape. While the hinge arms 5, 7 are described as being included within an inventory of hinge arms, it will be appreciated that other components of the hinge assembly can be selected from an inventory of components to design the movement of the hinge assembly. For instance, step 104 can include selecting gears attachable to the hinge arms from an inventory of gears.

Step 104 can include creating and/or using a computational model to determine the first and second profiles of the gears along the teeth based on the defined motion path. Step 104 can include obtaining information associated with the first and second profiles from the defined motion path. Obtaining such profile information can include reading data, analyzing data, transforming data, and/or processing data. Step 104 can include using iterative analysis techniques to determine the first and second profiles. Step 104 can include using curve fitting techniques to determine the first and second profiles. For instance, determining the first and second profiles can include using linear regression and/or nonlinear regression techniques. Step 104 can include fabricating the first and second profiles based on the defined motion path. Step 104 can include reconfiguring or varying the first and second profiles of the gears along their teeth based on the defined motion path. Step 104 can include varying the first and second profiles together such that at any specific or particular rotational position of the second hinge arm relative to the first hinge arm, the radius of the first gear and the radius of the second gear is generally constant.

Additionally, Fig. 6 shows that the method 100 can include a step 106 of connecting the first hinge arm to the second hinge arm such that the interaction between the first and second teeth along the first and second profiles translates the second hinge arm along the defined motion path as the second hinge arm rotates relative to the first hinge arm. Step 106 can include connecting the first hinge arm to the second hinge arm with a linkage. Step 106 can include connecting the first hinge arm to the second hinge arm for assembly, repair, and/or reconfiguration of the hinge assembly. In an embodiment, the hinge arms included in the inventory can have common connecting features. This beneficially facilitates assembling, repair, and/or reconfiguration of the hinge assembly by simply switching hinge arms in and out of the hinge assembly. Step 106 can include reconfiguring the first and second profiles of the gears along the teeth. Step 106 can include connecting a motion assist mechanism having a resilient member described below to the first hinge arm and the second hinge arm.

In an embodiment, one or more acts of the method described above may be performed by a computer system having at least one processing module configured to execute computer executable instructions and process operational data. The processing module may be operably coupled to a memory storing an application including computer executable instructions and operational data constituting a program to perform acts 102 and 104, and/or 106 of method 100. For instance, the processing module may be operably coupled to a memory storing an application including computer executable instructions and operational data comprising a program to determine the first and second profiles based on the defined motion path of the second hinge arm.

In other embodiments, one or more steps of method 100 may be repeated one or more times. For instance, if any issues arise or change is desired regarding the movement of the hinge assembly, the hinge arms can be removed, varied or reconfigured, and/or replaced with alternatives to make adjustments as needed.

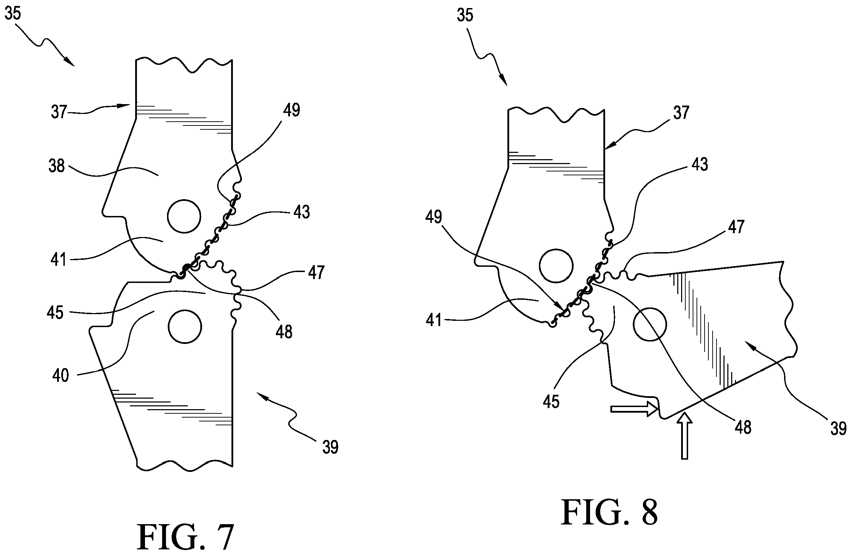

Figs. 7 and 8 show a hinge assembly 35 according to another embodiment. The hinge assembly 35 can be similar to the hinge assembly 3. For example, the hinge assembly 35 includes a first or upper hinge arm 37 and a second or lower hinge arm 39. A linkage or center linkage can be pivotally attached to the upper hinge arm 37 and to the lower hinge arm 39. The hinge assembly 35 can be made according to any of the methods described herein.

A distal portion 38 of the upper hinge arm 37 defines a first gear 41 having a first plurality of teeth 43. A proximal portion 40 of the lower hinge arm 39 defines a second gear 45 having a second plurality of teeth 47. The second teeth 47 of the second gear 45 are arranged to interact or mesh with the first teeth 43 of the first gear 41.

The profiles of the first and second gears 41, 45 along the teeth can follow any suitable shape but the first profile of the first gear 41 along the first teeth 43 is shown generally following a segment of an ellipse and the second profile of the second gear 45 along the second teeth 47 is shown generally following a segment of a circle. In other embodiments, at least one of the first profile or the second profile does not follow a traditional geometric shape but instead follows a free spline.

An ICoR 48 of the lower hinge arm 39 is generally located at a point of contact between the first and second teeth 43, 47. In use, the ICoR 48 of the lower hinge arm 39 moves as the point of contact between the first teeth 43 and the second teeth 47 changes, resulting in both rotational and translation movement of the lower hinge arm 39. As the lower hinge arm 39 rotates relative the upper hinge arm 37, the ICoR 48 of the lower hinge arm 39 moves along an ICoR path 49 as seen in Figs. 7 and 8.

The ICoR path 49 generally corresponds to the movement of the lower hinge arm 39 relative to the upper hinge arm 37 as the second teeth 47 of the second gear 45 moves along the first teeth 43 of the first gear 41.

In the illustrated embodiment, the ICoR path 49 extends along an arcuate line through the first teeth 43 of the first gear 41, generally corresponding to a segment of an ellipse. When a user's knee is flexed in flexion, the second teeth 47 on the second gear 45 advance over the teeth of the first gear, which, in turn, controls movement of the lower hinge arm 39 and shifts the lower hinge arm 39 in both the vertical and horizontal directions as it rotates relative to the upper hinge arm 37 as seen in Fig. 8.

Similar to the previously described embodiments, the profiles of the first and second gears 41, 45 along the first and second teeth 43, 47 can be selected or varied as needed to generate a complex hinge motion. The complex hinge motion can include a variable translation of the lower hinge arm 39 relative to the first hinge arm 37 along the ICoR path 49. By way of example, the profiles of the first and second gears 41, 45 along the first and second teeth 43, 47 can be selected or varied such that between about 0 and about 60 degrees flexion, the lower hinge arm 39 shifts in a positive vertical direction with generally no shift in a horizontal direction. From between about 60 and about 140 degrees flexion, the lower hinge arm 39 can shift in the positive vertical direction and a negative horizontal direction. From between about 140 and about 180 degrees flexion, the lower hinge arm 39 can shift in the negative vertical direction and in the negative horizontal direction.

According to a variation, the profiles of the first and second gears 41, 45 along the first and second teeth 43, 47 can be selected or varied such that between about 0 and about 60 degrees flexion, the lower hinge arm 39 shifts between about 4 mm and 6 mm in a positive vertical direction with generally no shift in a horizontal direction. From between about 60 and about 140 degrees flexion, the lower hinge arm 39 can shift between about 10 mm and about 12 mm in the positive vertical direction and between about 16 and 19 mm in the negative horizontal direction. From between about 140 and about 180 degrees flexion, the lower hinge arm 39 can shift between about 4 mm and 6 mm in the negative vertical direction and about 9 mm and 13 mm in the negative horizontal direction. It will be appreciated that the hinge assembly 35 can adjust the lower hinge arm 39 more or less in the vertical and horizontal directions.

In some embodiments, the profiles of the first and second gears 41, 45 along the first and second teeth 43, 47 can be selected or varied to shift the ICoR 48 of the lower hinge arm 39 backward (e.g., in a negative horizontal direction) during a period of flexion, generally simulating the anatomical movements of the knee. This beneficially allows the hinge assembly 35 to provide better support and comfort to a user. It will be appreciated that the profiles of the first and second gears 41, 45 along the first and second teeth 43, 47 can be selected or varied to shift the lower hinge arm 39 more or less, in different directions, at different angles, and/or in different patterns during flexion or extension.

In an embodiment, a defined motion path of the lower hinge arm 39 may first be selected by a clinician. The profiles of the first and second gears 41, 45 along the teeth 43, 47 can then be selected or adapted to generate a defined ICoR path 49 of the lower hinge arm 39 based on the defined motion path. This may include varying the profile of the gears 41, 45 along the teeth 43, 47 and/or a ratio or distance between the gears 41, 45 and the teeth 43, 47. The defined ICoR path 49 of the lower hinge arm 39 can then direct the movement of the lower hinge arm 39 along the defined motion path as the hinge assembly 35 moves through flexion and extension.

The interaction between the first and second gears 41, 45 thus allows the hinge assembly 35 to generate relatively complex movements through flexion and extension without the use of complicated components or designs as found in the prior art.

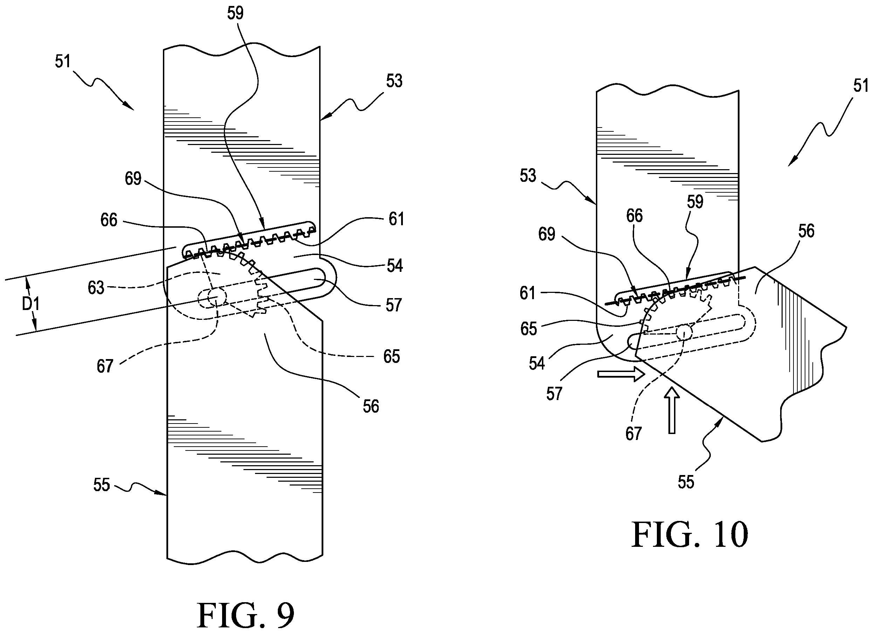

Figs. 9 and 10 show a hinge assembly 51 for use in an orthopedic device according to yet another embodiment. The hinge assembly 51 can be similar to the previously described hinge assembly embodiments. For instance, the hinge assembly 51 can include a first or upper hinge arm 53 and a second or lower hinge arm 55.

A distal portion 54 of the upper hinge arm 53 defines an elongate slot 57. A first gear 59 defining a first plurality of teeth 61 is located on the distal portion 54 a distance above the elongate slot 57. The first gear 59 can comprise a linear gear or rack. In other embodiments, the first gear 59 can be a free spline or curved gear.

The lower hinge arm 55 includes a second gear 63 comprising a pinion gear defining a plurality of second teeth 65 arranged to interact or mesh with the first teeth 61 of the first gear 59 on the upper hinge arm 53. The second gear 63 can be generally elliptical or circular with the first teeth 65 having an elongate configuration.

As seen, a fastener 67 such as a rivet can be attached to the lower hinge arm 55 and the second gear 63. The fastener 67 is slidably received within the slot 57 defined in the upper hinge arm 53. The fastener 67 and the first gear 59 are spaced from one another a distance D1. The distance D1 can be selected so that the first and second teeth 61, 65 mesh with one another during use.

The first and second gears 59, 63 can be integral to the upper and lower hinge arms 53, 55. In other embodiments, at least one of the first gear 59 or the second gear 63 can be removably attached to the upper hinge arm 53 or lower hinge arm 55, respectively. As described above, the gears 59, 63 can be selected from an inventory of gears for use in the hinge assembly 51. The gears included in the inventory can vary in material, profiles along the teeth, size, shape, teeth, combinations thereof, or other suitable characteristics.

An ICoR 66 of the lower hinge arm 55 is generally located at the point of contact between the first gear 59 and the second gear 63. In use, the ICoR 66 of the lower hinge arm 55 moves as the point of contact between the first teeth 61 and the second teeth 65 changes. More specifically, the ICoR 66 of the lower hinge arm 55 moves along an ICoR path 69 as the lower hinge arm 55 rotates about the upper hinge arm 53, as seen in Figs. 9 and 10.

The profiles of the first and second gears 59, 63 along the first and second teeth 61, 65 can be selected or varied to generate a complex hinge motion. The complex hinge motion can include a variable or non-linear translation of the lower hinge arm 55 relative to the upper hinge arm 53 along the ICoR path 69. In the illustrated embodiment, the ICoR path 69 extends along a generally linear line through the first teeth 61 on the first gear 59. When the knee of a user is flexed or in flexion, the interaction between the teeth 61, 65 translates the lower hinge arm 55 in both the vertical and horizontal directions as the lower hinge arm 55 rotates relative to the upper hinge arm 53. The fastener 67 attached to the second gear 63 to slide within the slot 57 with the translation of the lower hinge arm 55.

The magnitude and direction of translation of the lower hinge arm 55 as the hinge assembly 51 moves through flexion and extension can be selected to match anatomical knee motion and/or apply healing or corrective loads to the user's knee. For instance, the profiles of the first and second gears 59, 63 along the first and second teeth 61, 65 can be selected or varied such that between about 0 and about 30 degrees flexion, the lower hinge arm 55 shifts in a negative vertical direction and a positive horizontal direction. Between about 30 degrees and about 60 degrees flexion, the lower hinge arm 55 can shift in the positive vertical direction and in the positive horizontal direction.

Between about 60 and about 120 degrees flexion, the lower hinge arm 55 can shift in the positive vertical direction and in the negative horizontal direction. Between about 120 and about 180 degrees flexion, the lower hinge arm 55 can shift in the negative vertical direction and in the negative horizontal direction.

According to a variation, the profiles of the first and second gears 59, 63 along the first and second teeth 61, 65 can be selected or varied such that between about 0 degrees and about 30 degrees flexion, the lower hinge arm 55 shifts between about 1 mm and about 3 mm in a negative vertical direction and between about 2 mm and about 4 mm in a positive horizontal direction. Between about 30 and about 60 degrees flexion, the lower hinge arm 55 can shift about 1 and about 33 mm in the positive vertical direction and between about 1 mm and about 3 mm in the positive horizontal direction. Between about 60 and 120 degrees flexion, the lower hinge arm 55 can shift about 10 mm in the positive vertical direction and between about 5 mm and about 7 mm in the negative horizontal direction. Between about 120 and 180 degrees flexion, the lower hinge arm 55 can shift between about 6 mm and about 9 mm in the negative vertical direction and between about 13 mm and about 16 mm in the negative horizontal direction.

The hinge assembly 51 can thus beneficially generate complex hinge motion using a simple and intuitive construction. It will be appreciated that the hinge assembly 51 can be adapted such that the lower hinge arm 55 shifts more or less, in different directions, at different angles, and/or in different patterns during flexion and/or extension.

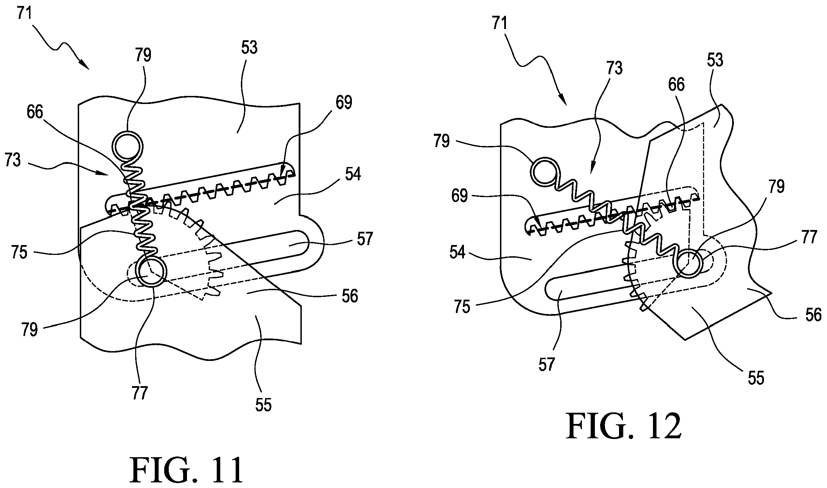

Figs. 11 and 12 show a hinge assembly 71 according to yet another embodiment. The hinge assembly 71 can be similar to the hinge assembly 51 except that it includes a motion assist mechanism 73. In an embodiment, the motion assist mechanism 73 can include a resilient member 75 such as a linear elastic spring member connected between the upper and lower hinge arms 53, 55. For instance, the resilient member 75 can include a first end 77 connected to the fastener 67 and a second end 79 connected to the upper hinge arm 53. The first and second ends 77, 79 can be respectively connected to the fastener 67 and the upper hinge arm 53 in any suitable manner.

In flexion or when the knee is brought from an extension position (shown in Fig. 11) to the flexion position (shown in Fig. 12), the interaction between the first and second teeth 61, 65 rotates and translates the lower hinge arm 55 relative to the upper hinge arm 53. Because the spring member 75 is connected to both the upper and lower hinge arms 53, 55, this movement pulls the first end 77 of the spring member 75 away from the second end 79 of the resilient member 75, stretching the spring member 75.

The stored mechanical energy in the spring member 75 then biases the spring member 75 back toward its equilibrium position, which pulls the first end 77 back toward the second end 79 of the resilient member 75. This restoring force can help control movement of the hinge assembly 71 from extension to flexion. It also can help urge the lower hinge arm 55 to reverse its flexion movement around the upper hinge arm 53, which forces the hinge assembly 71 back toward the extension position.

It will be appreciated that in other embodiments, the motion assist mechanism 73 can be arranged to create flexion assist or to decelerate extension of the knee as it approaches a set angle. In addition, the resilient member 75 can be selected based on one or more properties (e.g., stiffness) to achieve a desired load on the lower hinge arm 55. In other embodiments, the spring member 75 can be arranged to exert a first biasing force toward extension when the hinge assembly arrives at a first flexion angle and to exert a second biasing force different from the first when the hinge assembly arrives at or approaches a second flexion angle. In other embodiments, the motion assist mechanism can include a plurality of resilient members to exert biasing forces on the lower hinge arm toward the flexion position and/or the extension position.

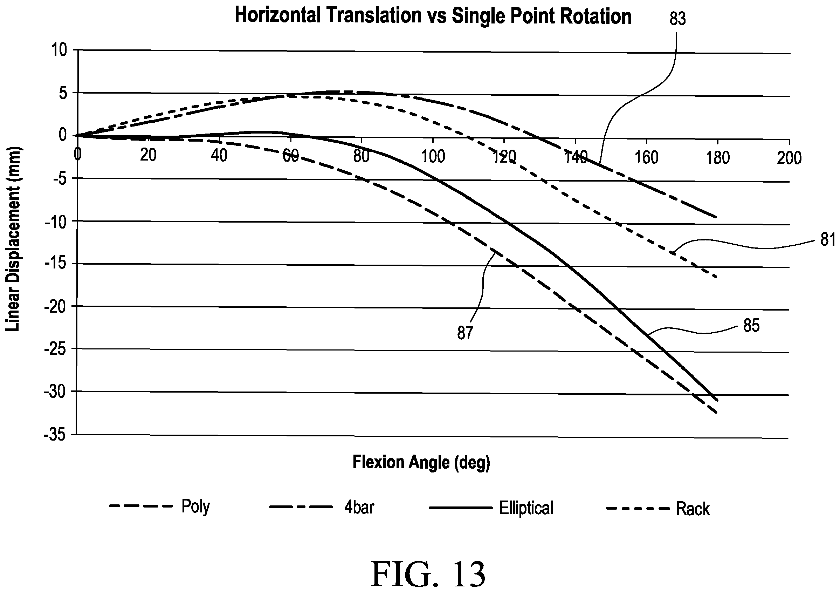

It will be appreciated that the hinge assembly embodiments described herein are adapted to better and more easily generate complex hinge motion than in the prior art. Moreover, the profiles of the gears along the teeth can be varied or selected to generally match anatomical knee motion and/or to apply loads to the knee. For instance, Fig. 13 illustrates horizontal displacement data taken and plotted at different flexion angles. Curve 81 of a hinge assembly embodiment having a rack gear design (e.g., the hinge assembly 51) produces similar horizontal translation as the curve of a prior art 4-bar hinge seen in curve 83. The path of curve 81 also generally follows the path of the curve 83.

Also shown, the curve 85 of a hinge assembly embodiment having an elliptical gear design (e.g., the hinge assembly 35) produces similar horizontal translation as the curve of a traditional polycentric hinge seen in curve 87. The path of curve 85 also generally follows the path of the curve 87.

The hinge assembly embodiments can thus be adapted to generate horizontal displacement similar to the more complicated 4-bar and polycentric hinges without the use of complex and more breakable components as in the prior art.

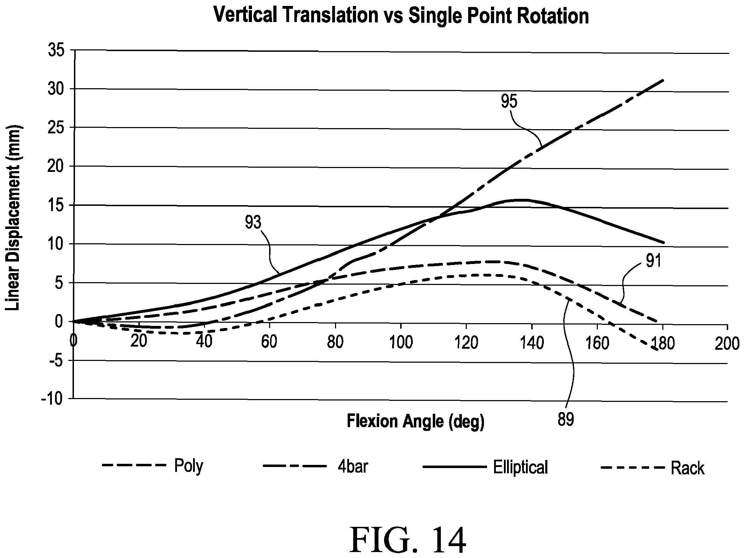

Fig. 14 illustrates vertical displacement data taken and plotted at different flexion angles. Curve 89 of a hinge assembly having a rack and pinon design produces similar vertical translation as the curve of a traditional polycentric hinge seen in curve 91. The path of curve 89 also generally follows the path of the curve 91. Curve 93 of the hinge assembly having an elliptical gear design produces more vertical translation between about 0 and 110 degrees flexion than the curve of a traditional 4-bar hinge seen in curve 95. The path of the curve 93 generally follows the path of curve 95 between about 0 and 110 degrees and departs from the curve 95 between about 110 and 180 degrees. The hinge assembly embodiments can thus also be made or adapted to generate relatively complex hinge motion for different applications.

The embodiments described herein are to be regarded as exemplary only, as any hinge assembly is possible. For instance, while the second hinge arm is described as a lower hinge arm, in other embodiments, the second hinge arm can be an upper hinge arm. Thus, an upper hinge arm can be rotatable relative to the lower hinge arm. In other embodiments, the upper and lower hinge arms can both rotate relative to one another. In yet other embodiments, while the first embodiment is shown including a rotation stop, it should be appreciated that any of the hinge assembly embodiments can include one or more rotation stops. In other embodiments, the upper hinge arm can define a gear having an elliptical or irregular geometric shape.

In other embodiments, any of the hinge assembly embodiments can include a motion assist mechanism to create flexion assist, extension assist, or the like. In yet other embodiments, the motion assist mechanism can include two, three, four, or any other number of resilient members. In other embodiments, the upper hinge arm can rotate relative to the lower hinge arm.

While one hinge assembly is described, it should appreciated that the embodiments can include a pair of hinges located near the knee. In yet other embodiments, an orthopedic device can include a first hinge assembly on the medial side and a second hinge assembly on the lateral side. The first and second hinge assemblies can have different designs such that a desired rotation of the user's tibia can be achieved.

As discussed above, the hinge assembly embodiments can be arranged and oriented to create a desired anatomical correction. For example, the hinge assembly can connect to upper and lower cuffs. The upper cuff secures to the upper leg, such as over the thigh, and the lower cuff secures to the lower leg, such as over the shin or calf. Through the ICoR of the lower hinge arm, the lower cuff can be arranged and oriented so as to exert an anteriorly directed force onto the lower leg to create an anatomical correction.

While various aspects and embodiments have been disclosed herein, other aspects and embodiments are contemplated.

## Claims

1. A method (100) of making a hinge assembly, the method comprising the following steps of:
selecting a defined motion path (27) of a hinge assembly (3) comprising a first hinge arm (5) including a first gear (19) having a first plurality of teeth (21), and a second hinge arm (7) including a second gear (23) having a second plurality of teeth (25) arranged to mesh with the first teeth (21), the defined motion path (27) during use including a translation of the second hinge arm (7) relative to the first hinge arm (5);
determining a first profile of the first gear (19) along the first teeth (21) and a second profile of the second gear (23) along the second teeth (25) based on the defined motion path (27); and
connecting the first hinge arm (5) to the second hinge arm (7) such that as the second hinge arm (7) during use rotates relative to the first hinge arm (5) the interaction between the first and second teeth (21, 25) along the first and second profiles translates the second hinge arm (7) along the defined motion path (27),
**characterized in that** the first profile is different from the second profile such that the translation during use is a variable translation which occurs **in that** second hinge arm (7) has a different motion relative to the first hinge arm (5), and the defined motion path (27) comprises an instantaneous center of rotation path (27) of the second hinge arm (7) located at or near the first teeth (21) of the first gear (19).

2. The method (100) of claim 1, **characterized in that** the first profile follows a linear line and the second profile follows a segment of a circle.

3. The method (100) of any one of the preceeding claims, **characterized in that** at least one of the first profile and the second profile follows a free spline.

4. The method (100) of any one of the preceding claims, **characterized in that** determining the first and second profiles based on the defined motion path (27) includes selecting the first and second gears (19, 23) from an inventory of gears having different profiles.

5. The method (100) of claim 1, **characterized in that** the instantaneous center of rotation path (27) generally corresponds to anatomical knee motion of a user.

6. The method (100) of claim 1, **characterized in that** the instantaneous center of rotation path (27) deviates from anatomical knee motion of a user.

7. The method (100) of claim 1, **characterized in that** the instantaneous center of rotation path (27) of the second hinge arm (7) extends along a linear line and an arcuate line.

8. The method (100) of claim 1, **characterized in that** the defined motion path (27) comprises the second hinge arm (7) translating in both a horizontal direction toward the femoral plane of a user and a horizontal direction away from the femoral plane between about 0 degrees and about 60 degrees flexion of a knee of a user.

9. The method (100) of claim 1, **characterized in that** the defined motion path (27) comprises the second hinge arm (7) translating in both a vertical direction away from a center of a knee of a user and a vertical direction toward the knee center between about 0 and about 60 degrees flexion of the knee of the user.

10. The method (100) of claim 1, **characterized in that** the defined motion path (27) comprises the second hinge arm (7) translating in both a vertical direction away from a center of a knee of a user and a vertical direction toward the knee center between about 60 and about 160 degrees flexion of the knee of the user.

11. The method (100) of any one of the preceding claims, **characterized in** further comprising connecting a resilient member (75) to the first hinge arm (5) and the second hinge arm (7).

12. The method (100) of claim 11, **characterized in that** the resilient member (75) is arranged to bias the hinge assembly (3) toward an extension position when the hinge assembly (3) reaches a selected flexion angle.

13. The method (100) of any one of the preceding claims, **characterized in that** the hinge assembly (3) is adapted for use with a prosthetic device.

## Patentansprüche

1. Ein Verfahren (100) zum Herstellen einer Gelenkstückbaugruppe, wobei das Verfahren die folgenden Schritte umfasst:
Auswählen eines definierten Bewegungswegs (27) einer Gelenkstückbaugruppe (3), umfassend einen ersten Gelenkstückarm (5), der ein erstes Zahnrad (19) beinhaltet, das eine erste Vielzahl von Zähnen (21) aufweist, und einen zweiten Gelenkstückarm (7), der ein zweites Zahnrad (23) beinhaltet, das eine zweite Vielzahl von Zähnen (25) aufweist, die derart angeordnet sind, um mit den ersten Zähnen (21) ineinanderzugreifen, wobei der definierte Bewegungsweg (27) während dem Gebrauch eine Übersetzung des zweiten Gelenkstückarms (7) bezüglich des ersten Gelenkstückarms (5) beinhaltet;
Bestimmen eines ersten Profils des ersten Zahnrads (19) entlang der ersten Zähne (21) und eines zweiten Profils des zweiten Zahnrads (23) entlang der zweiten Zähne (25), basierend auf dem definierten Bewegungsweg (27); und
Verbinden des ersten Gelenkstückarms (5) mit dem zweiten Gelenkstückarm (7), sodass während sich der zweite Gelenkstückarm (7) im Gebrauch bezüglich des ersten Gelenkstückarms (5) dreht, die Wechselwirkung zwischen den ersten und zweiten Zähnen (21, 25) entlang der ersten und zweiten Profile den zweiten Gelenkstückarm (7) entlang des definierten Bewegungswegs (27) übersetzt,
**dadurch gekennzeichnet, dass** sich das erste Profil von dem zweiten Profil derart unterscheidet, sodass die Übersetzung im Gebrauch eine variable Übersetzung ist, die stattfindet, indem der zweite Gelenkstückarm (7) eine andere Bewegung bezüglich des ersten Gelenkstückarms (5) aufweist und der definierte Bewegungsweg (27) einen unmittelbaren Drehmittelpunktweg (27) des zweiten Gelenkstückarms (7) umfasst, der sich an den oder nahe der ersten Zähne (21) des ersten Zahnrads (19) befindet.

2. Das Verfahren (100) nach Anspruch 1, **dadurch gekennzeichnet, dass** das erste Profil einer linearen Linie folgt und das zweite Profil einem Segment eines Kreises folgt.

3. Das Verfahren (100) nach einem der vorstehenden Ansprüche, **dadurch gekennzeichnet, dass** mindestens eines von dem ersten Profil und dem zweiten Profil einer freien Keilwelle folgt.

4. Das Verfahren (100) nach einem der vorstehenden Ansprüche, **dadurch gekennzeichnet, dass** das Feststellen der ersten und zweiten Profile basierend auf dem definierten Bewegungspfad (27) das Auswählen der ersten und zweiten Zahnräder (19, 23) aus einem Vorrat von Zahnrädern mit unterschiedlichen Profilen beinhaltet.

5. Das Verfahren (100) nach Anspruch 1, **dadurch gekennzeichnet, dass** der unmittelbare Drehmittelpunktweg (27) im Allgemeinen mit einer anatomischen Kniebewegung eines Anwenders übereinstimmt.

6. Das Verfahren (100) nach Anspruch 1, **dadurch gekennzeichnet, dass** der unmittelbare Drehmittelpunktweg (27) von einer anatomischen Kniebewegung eines Anwenders abweicht.

7. Das Verfahren (100) nach Anspruch 1, **dadurch gekennzeichnet, dass** sich der unmittelbare Drehmittelpunktweg (27) des zweiten Gelenkstückarms (7) entlang einer linearen Linie und einer bogenförmigen Linie erstreckt.

8. Das Verfahren (100) nach Anspruch 1, **dadurch gekennzeichnet, dass** der definierte Bewegungsweg (27) das Übersetzen des zweiten Gelenkstückarms (7) sowohl in eine horizontale Richtung zur Oberschenkelebene eines Anwenders, als auch in eine horizontale Richtung weg von der Oberschenkelebene um zwischen etwa 0 Grad und etwa 60 Grad an Biegung eines Knies eines Anwenders umfasst.

9. Das Verfahren (100) nach Anspruch 1, **dadurch gekennzeichnet, dass** der definierte Bewegungsweg (27) das Übersetzen des zweiten Gelenkstückarms (7) sowohl in eine vertikale Richtung weg von einer Kniemitte eines Anwenders, als auch in eine vertikale Richtung hin zur Kniemitte um zwischen etwa 0 Grad und etwa 60 Grad an Biegung des Knies eines Anwenders umfasst.

10. Das Verfahren (100) nach Anspruch 1, **dadurch gekennzeichnet, dass** der definierte Bewegungsweg (27) das Übersetzen des zweiten Gelenkstückarms (7) sowohl in eine vertikale Richtung weg von einer Kniemitte eines Anwenders, als auch in eine vertikale Richtung hin zur Kniemitte um zwischen etwa 60 Grad und etwa 160 Grad an Biegung des Knies eines Anwenders umfasst.

11. Das Verfahren (100) nach einem der vorstehenden Ansprüche, **dadurch gekennzeichnet, dass** es weiter das Verbinden eines elastischen Elements (75) mit dem ersten Gelenkstückarm (5) und dem zweiten Gelenkstückarm (7) umfasst.

12. Das Verfahren (100) nach Anspruch 11, **dadurch gekennzeichnet, dass** das elastische Element (75) derart angeordnet ist, um die Gelenkstückbaugruppe (3) zu einer ausgezogenen Position hin vorzuspannen, wenn die Gelenkstückbaugruppe (3) einen ausgewählten Biegungswinkel erreicht hat.

13. Das Verfahren (100) nach einem der vorstehenden Ansprüche, **dadurch gekennzeichnet, dass** die Gelenkstückbaugruppe (3) zur Verwendung mit einer Prothesenvorrichtung angepasst ist.

## Revendications

1. Une procédé (100) de fabrication d'un ensemble formant charnière, le procédé comprenant les étapes suivantes consistant à :
choisir une trajectoire (27) de mouvement définie d'un ensemble formant charnière (3) comprenant un premier bras de charnière (5) incluant un premier engrenage (19) présentant une première pluralité de dents (21), et un second bras de charnière (7) incluant un second engrenage (23) présentant une seconde pluralité de dents (25) agencées de manière à s'engrener avec les premières dents (21), la trajectoire (27) de mouvement définie en cours d'utilisation incluant un mouvement de translation du second bras de charnière (7) par rapport au premier bras de charnière (5) ;
déterminer un premier profil du premier engrenage (19) le long des premières dents (21) et un second profil du second engrenage (23) le long des secondes dents (25) sur la base de la trajectoire (27) de mouvement définie ; et
relier le premier bras de charnière (5) au second bras de charnière (7) de sorte que lorsque le second bras de charnière (7) en cours d'utilisation pivote par rapport au premier bras de charnière (5), l'interaction entre les premières et secondes dents (21, 25) le long des premier et second profils permet un mouvement de translation du second bras de charnière (7) le long de la trajectoire (27) de mouvement définie,
**caractérisé en ce que** le premier profil est différent du second profil de sorte que le mouvement de translation en cours d'utilisation est un mouvement de translation variable qui se produit **en ce que** le second bras de charnière (7) présente un mouvement différent par rapport au premier bras de charnière (5), et la trajectoire (27) de mouvement définie comprend un centre instantané de la trajectoire (27) de rotation du second bras de charnière (7) situé sur ou à proximité des premières dents (21) du premier engrenage (19).

2. La procédé (100) selon la revendication 1, **caractérisé en ce que** le premier profil suit une ligne linéaire et le second profil suit un segment d'un cercle.

3. La procédé (100) selon l'une quelconque des revendications précédentes, **caractérisé en ce qu'**au moins l'un parmi le premier profil et le second profil suit une cannelure libre.

4. La procédé (100) selon l'une quelconque des revendications précédentes, **caractérisé en ce que** la détermination des premier et second profils sur la base de la trajectoire (27) de mouvement définie inclut la sélection des premier et second engrenages (19, 23) à partir d'une liste d'engrenages présentant des profils différents.

5. La procédé (100) selon la revendication 1, **caractérisé en ce que** le centre instantané de la trajectoire (27) de rotation correspond généralement à un mouvement anatomique du genou d'un utilisateur.

6. La procédé (100) selon la revendication 1, **caractérisé en ce que** le centre instantané de la trajectoire (27) de rotation dévie le mouvement anatomique du genou d'un utilisateur.

7. La procédé (100) selon la revendication 1, **caractérisé en ce que** le centre instantané de la trajectoire (27) de rotation du second bras de charnière (7) s'étend le long d'une ligne linéaire et d'une ligne arquée.

8. La procédé (100) selon la revendication 1, **caractérisé en ce que** la trajectoire (27) de mouvement définie comprend le second bras de charnière (7) effectuant un mouvement de translation à la fois dans une direction horizontale vers le plan fémoral d'un utilisateur et une direction horizontale s'éloignant du plan fémoral entre environ 0 degrés et environ 60 degrés de flexion d'un genou d'un utilisateur.

9. La procédé (100) selon la revendication 1, **caractérisé en ce que** la trajectoire (27) de mouvement définie comprend le second bras de charnière (7) effectuant un mouvement de translation à la fois dans une direction verticale s'éloignant d'un centre d'un genou d'un utilisateur et une direction verticale vers le centre du genou entre environ 0 et environ 60 degrés de flexion du genou de l'utilisateur.

10. La procédé (100) selon la revendication 1, **caractérisé en ce que** la trajectoire (27) de mouvement définie comprend le second bras de charnière (7) effectuant un mouvement de translation à la fois dans une direction verticale s'éloignant d'un centre d'un genou d'un utilisateur et une direction verticale vers le centre du genou entre environ 60 et environ 160 degrés de flexion du genou de l'utilisateur.

11. La procédé (100) selon l'une quelconque des revendications précédentes, **caractérisé en ce qu'**il comprend en outre la liaison d'un élément élastique (75) au premier bras de charnière (5) et au second bras de charnière (7).

12. La procédé (100) selon la revendication 11, **caractérisé en ce que** l'élément élastique (75) est agencé de manière à solliciter l'ensemble formant charnière (3) vers une position d'extension lorsque l'ensemble formant charnière (3) atteint un angle de flexion choisi.

13. La procédé (100) selon l'une quelconque des revendications précédentes, **caractérisé en ce que** l'ensemble formant charnière (3) est conçu pour être utilisé avec un dispositif prothétique.
